# EUROPEAN PATENT APPLICATION

(11) **EP 4 672 259 A1**
(43) Date of publication of application: **31.12.2025**
(21) Application number: 24020314.1
(22) Date of filing: 14.10.2024
(51) Int. Cl.: G16H 40/67

(54) **METHOD AND SYSTEM FOR MONITORING OF ELDERLY PEOPLE**

(30) Priority: 28.06.2024 EP 24020217
(71) Applicant: Onemeter Spolka z Ograniczona Odpowiedzialnoscia, 20-262 Lublin (PL)
(72) Inventor: Brzozowski, Mateusz, 00-739 Warszawa (PL); Janicki, Artur, 05-501 Piaseczno (PL)
(74) Representative: Marcinska-Porzuc, Aleksandra

(57) **Abstract**

The invention relates to a method of monitoring human well-being, in which the recording of activity undertaken by the monitored person is used, and measurement data is transmitted to a remote device, which is distinguished by the fact that it includes non-invasive reading of the consumption of at least one utility present at the monitored person's location, and comprises the steps of: transmitting read data on the amount of at least one consumed utility to a master unit, directly via a telecommunications network or using an intermediary communication device, storing the read data on the amount of at least one consumed utility in a database, performing an analysis of the read data on the amount of at least one consumed utility in a given time, and transmitting, to a monitoring person via a telecommunications network, the read data and/or the results of the performed analysis on the typical or atypical consumption by the monitored person of at least one mentioned utility. The invention also relates to a system in which the mentioned method is implemented.

## Description

### Field of the invention

This invention relates to a method and system for non-invasive remote monitoring of a person's well-being at her/his place of residence based on her/his consumption of utilities such as electricity, water, and gas.

### State of the art

There are various methods and systems for remote monitoring of human well-being. For example, visual and audio monitoring systems are used, which involve installing devices such as motion sensors, cameras, and microphones in the monitored person's residence. These devices are intended to check how the monitored person moves around the apartment, for example, whether she/he has gotten out of bed, opened a medicine cabinet, walked from the room to the kitchen, and so on. In turn, the microphone is intended to record disturbing sounds that may indicate, for example, the monitored person falling due to fainting or other ailments. Another method is to equip the monitored person with various sensors, such as a wristband, smartwatch, or sensors mounted on the chest. US20210015415A1 discloses methods of monitoring human activity and inactivity based on analyzing activities such as performed gestures, walking, drinking, taking medications, eating and taking food, and/or sleeping as well as falls. In such methods, the vital functions of the monitored person's body, such as pulse or oxygen saturation, are also monitored. CA2509279A1 discloses systems for monitoring medication intake by patients. Examples of similar methods of monitoring human well-being are disclosed, for example, in US20230091003A1, EP3593264, or WO2016057564A1. There are also systems that analyze electricity consumption by placing a suitable probe in the user's electrical installation, which measures energy independently of the measurement performed by the electricity supplier.

However, the above-mentioned methods of monitoring human well-being have several disadvantages. For example, they require the installation of devices that may violate the privacy of the monitored person, such as sensors, cameras, or microphones, and are therefore often unacceptable to those people who are monitored. Other methods require the monitored person to wear devices such as wristbands or sensors on her/his body, which is also inconvenient and often unacceptable. Also, the monitored person may remove the wristband, for example, before bathing and forget to put it back on. Furthermore, these devices require regular charging. After discharging, they stop working, which can cause interruptions in monitoring and resulting consequences, such as concern for the observer or lack of an alarm in the event of a disturbing incident. Finally, other existing methods require interference with the electrical installation at the monitored person's residence, which is associated with costs, the need to involve a person with appropriate qualifications, and may also not be accepted by the monitored person.

Therefore, the primary object of this invention is to enable non-invasive remote monitoring of human well-being that does not require the person to wear any devices, nor interference with installations at the monitored person's residence, nor the installation of devices that may violate the privacy of the monitored person, such as sensors, cameras, or microphones. The invention uses the measurement of consumption of at least one utility at the monitored person's place of residence, carried out by at least one metering apparatus installed by the supplier of the specific utility. The invention provides notification to the monitoring person about the well-being of the monitored person via a telecommunications network by informing about typical or atypical consumption of at least one utility by the monitored person.

According to the invention, this object has been achieved by the features of independent claims 1 and 11, which relate to the method and the system using this method. This solution involves the possibility of device communication with utility metering apparatuses, for example, using an optical interface, reading a flashing diode, or another communication interface transmitting information about utility usage.

### Summary of the Invention

The invention relates to a method of monitoring human well-being, wherein the method involves indirect recording of the monitored person's activity through non-invasive reading of the consumption of at least one utility and is characterized in that it comprises the steps of:
- reading and transmitting the read data on the amount of at least one consumed utility to a master unit, directly via a telecommunications network or using an intermediary communication device,
- storing the read data in a dedicated utility consumption database, enabling the storage of current and historical data for analysis purposes,
- performing an analysis of the read data on the amount of at least one consumed utility in a given time by analytical means,
- transmitting the read data and/or the results of the performed analysis of typical or atypical consumption of at least one mentioned utility by the monitored person to the monitoring person via a telecommunications network.

The read utility is electricity and/or gas and/or water. The analysis of the read data on the amount of consumed utilities in a given time consists of comparing the current consumption of each utility with its own previously read historical data.

The read data on the amount of consumed utilities are transmitted via a telecommunications network to a remote server directly or using an intermediary communication device.

The read data on the remote server are stored in a database.

Analytical means operating on the server create a mathematical model of the consumption of mentioned utilities based on historical data relating to previous consumption, and based on the analysis of utility consumption, analysis results and optionally alerts are generated, which are transmitted via a telecommunications network to the monitoring person and presented on her/his terminal device. The analytical means create different mathematical models for the monitored person, depending on different times of the year, in which the characteristics of utility consumption may differ.

Reading the amount of consumed utility by the reading device is performed recurrently by connecting to the utility meter every predetermined time, for example, every 15 minutes, or on request from the server or intermediary device. Optionally, reading the amount of consumed utility by the reading device is performed continuously, and the data is transmitted after reading a measurement value that differs from the previously read value by a predetermined threshold value. Non-invasive reading of the amount of consumed utility is performed by reading the metering apparatus readings using its optical port or by counting light pulses generated by an optical light source integrated with the utility metering apparatus. Alternatively, reading the amount of consumed utility is performed by reading the metering apparatus readings using an isolated galvanic interface or a P1 port integrated with the utility metering apparatus. Preferably, the data transmission from the reading device is encrypted, for example using the AES protocol, to increase data transmission security.

The invention also relates to a system for monitoring human well-being, which includes at least one utility consumption metering apparatus, wherein the utility is consumed by the monitored person, and at least one device intended for transmitting read data from mentioned utility consumption metering apparatus to a master device, and a system for processing and storing read data, and is characterized in that the first reading device (DEVICE) is connected to the first utility metering apparatus (METER) in a non-invasive fashion, and provides reading of data on the consumption of the first utility and transmission of the read data to the master device (SERVER) or via a communication device (COMM. DEVICE) to the master device (SERVER).

Furthermore, the present system is distinguished by the fact that it has at least one second reading device (DEVICE) connected to the second utility metering apparatus (METER) in a non-invasive fashion, and providing reading of data on the consumption of the second utility, and transmitting the read data to the master device (SERVER) or via a communication device (COMM. DEVICE) to the master device (SERVER). Optionally, the system also has a third reading device (DEVICE) connected to the third utility metering apparatus (METER) operating in a non-invasive fashion, and providing reading of data on the consumption of the third utility and transmitting the read data to the master device (SERVER) or via a communication device (COMM. DEVICE) to the master device (SERVER).

All reading devices (DEVICE) have means for non-invasively reading or counting the amount of consumed utility and are connected to the master device (SERVER) in a manner ensuring data transmission. The master device (SERVER) has a system for processing the read data on the consumption of all read utilities, memory means (MEM) configured to store the read data on utility consumption, and means for performing computational operations, and means for transmitting the results of the analysis on the well-being of the monitored person to the monitoring person.

The first utility is electricity, the second utility is gas, and the third utility is water, or the first utility is electricity, the second utility is water, and the third is gas, or the first utility is water, the second utility is electricity, and the third utility is gas, or the first utility is water, the second utility is gas, and the third utility is electricity, or the first utility is gas, the second utility is electricity, and the third utility is water, or the first utility is gas, the second utility is water, and the third utility is electricity.

The master device is a server, the system for processing read data, and the means for performing computational operations is a computer program running on the server, and a database is implemented in the memory means, storing historical data on previous utility consumption. The reading device has means in the form of an optical port for non-invasively reading the amount of consumed utility or means for counting light pulses generated by an optical light source integrated with the utility metering apparatus.

Alternatively, the reading device has means in the form of an isolated galvanic interface or a P1 port integrated with the utility metering apparatus for non-invasively reading the amount of consumed utility.

### Brief description of the drawings

The subject of the invention is presented in embodiments in the drawings, where:
Fig. 1 illustrates a system for monitoring the well-being of a monitored person according to the invention, based on reading one utility consumed by said person,
Fig. 2 illustrates a system for monitoring the well-being of a monitored person according to the invention, based on reading three different utilities consumed by said person,
Fig. 3 illustrates in a block diagram a device according to the invention for reading the utility consumed by the monitored person,
Fig. 4 illustrates in a block diagram an exemplary embodiment of a master device for monitoring the well-being of a monitored person according to the invention.

### Detailed description of the embodiments according to the Invention

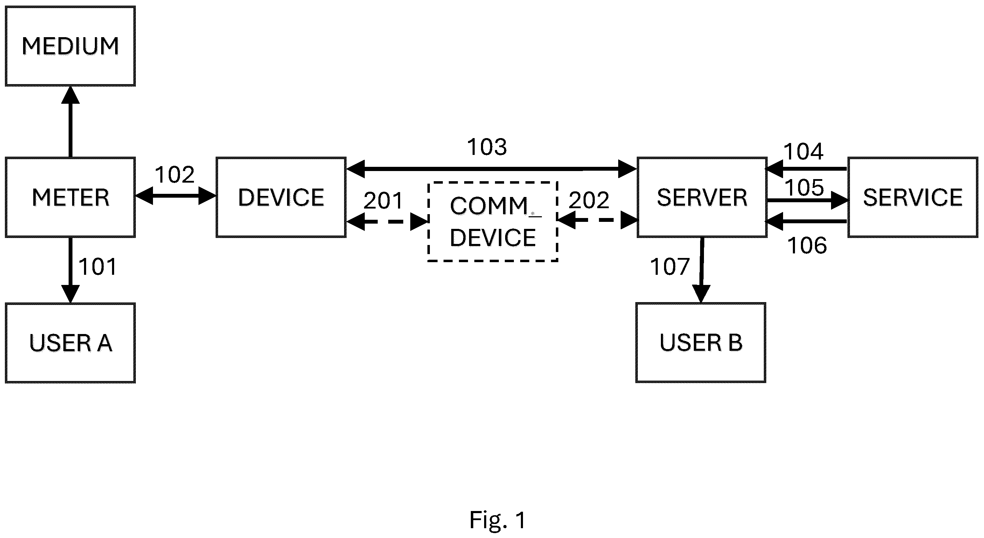

In the first aspect of this invention, a method of monitoring human well-being is presented using the measurement of consumption of one of many utilities, such as electricity, water, or gas. FIG. 1 shows a method and system according to the invention for monitoring human well-being based on reading the consumption of one utility, MEDIUM, for example, electricity. The energy metering apparatus, METER, in step 101, measures the consumption of electricity, MEDIUM, by the monitored person, USER A. Next, in step 102, the reading device, DEVICE, reads the amount of electricity, MEDIUM, consumed by the monitored user, USER A. The reading device, DEVICE, transmits, in step 103, data on the amount of consumed electricity, MEDIUM, to the remote master device, SERVER. Alternatively, the reading device, DEVICE, transmits, in step 201, data on the amount of consumed electricity, MEDIUM, to the intermediary communication device, COMM_DEVICE, and then the intermediary communication device, COMM _DEVICE, in step 202, transmits this data to the master device, SERVER.

Next, the master device, SERVER, stores the read data on the amount of consumed electricity, MEDIUM, in a database. The service, SERVICE, queries, in step 104, the master device, SERVER, for data on the amount of electricity, MEDIUM, consumed by the monitored user, USER A, in a specific time. The master device, SERVER, reads the requested data from the database and transmits, in step 105, the necessary data to the service, SERVICE. Then, based on this data, the service, SERVICE, using computational methods, analyzes the amount of electricity, MEDIUM, consumed by the monitored person, USER A. The results of the analysis performed by the service, SERVICE, for example, information about atypical consumption of electricity, MEDIUM, by the monitored person, USER A, is transmitted in step 106 to the master device, SERVER, and then in step 107 is transmitted to the monitoring person, USER B. In this way, the subject invention enables non-invasive remote monitoring of the well-being of the monitored user, USER A, by the monitoring user, USER B.

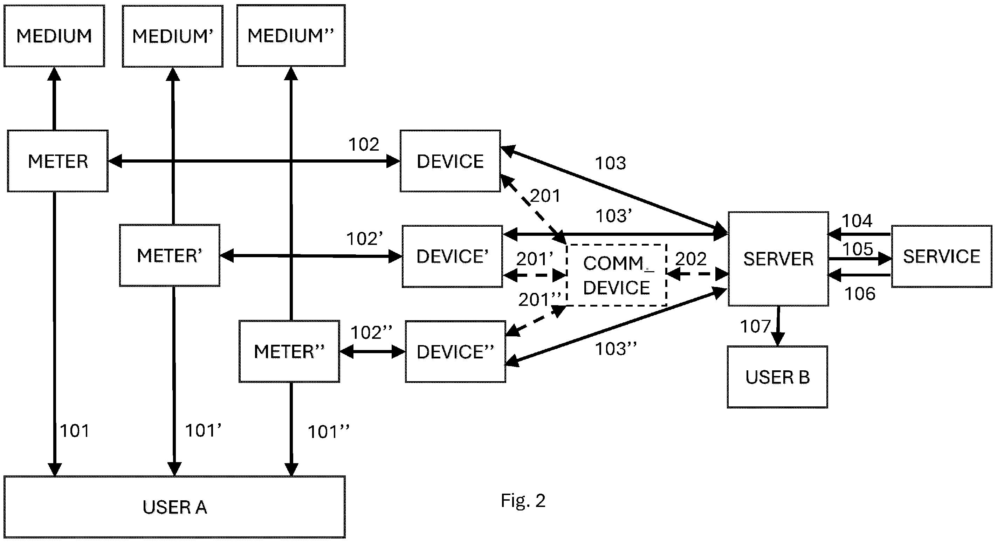

FIG. 2 illustrates a method and system for monitoring the well-being of a human, the monitored person, USER A, by the monitoring person, USER B, based on reading the consumption of more than one utility, for example, electricity, water, and gas. The first metering apparatus, METER, measures, in step 101, the consumption of the first utility in the form of electricity, MEDIUM, by the monitored person, USER A. The second metering apparatus, METER', measures, in step 101', the consumption of the second utility in the form of water, MEDIUM', by the monitored person, USER A. The third metering apparatus, METER", measures, in step 101", the consumption of the third utility in the form of gas, MEDIUM", by the monitored person, USER A. Then, the first reading device, DEVICE, reads, in step 102, the amount of the first utility, MEDIUM, consumed by the monitored person, USER A. The second device, DEVICE', reads, in step 102', the amount of the second utility, MEDIUM', consumed by the monitored person, USER A. The third device, DEVICE", reads, in step 102", the amount of the third utility, MEDIUM", consumed by the monitored person, USER A.

The reading devices, DEVICE, DEVICE', and DEVICE", transmit, in steps 103, 103', and 103", respectively, data on the amount of consumed utilities, MEDIUM, MEDIUM', and MEDIUM", respectively, to the master device, SERVER. Alternatively, the above reading devices transmit the read data on the amount of consumed utilities to the intermediary communication device, COMM _DEVICE, and then COMM_DEVICE transmits, in step 202, the read data to the server, SERVER.

Next, the master device, SERVER, stores the read data on the amount of consumed utilities, MEDIUM, MEDIUM', and MEDIUM", in a database. The service, SERVICE, queries, in step 104, the master device, SERVER, for data on the amount of utilities, MEDIUM, MEDIUM', and MEDIUM", consumed by the monitored person, USER A, in a specific time. The master device, SERVER, reads this data from the database and transmits it, in step 105, to the service, SERVICE. Then, based on this information, the service, SERVICE, using computational methods, analyzes the amount of consumed utilities, MEDIUM, MEDIUM', and MEDIUM" by the monitored person, USER A. The results of this analysis, for example, information about atypical consumption of utilities, MEDIUM, MEDIUM', and MEDIUM", by the monitored person, USER A, is transmitted, in step 106, to the master device, SERVER, and then is transmitted, in step 107, to the monitoring person, USER B. Thus, the proposed solution enables non-invasive, remote monitoring of the well-being of the monitored person, USER A, by the monitoring person, USER B.

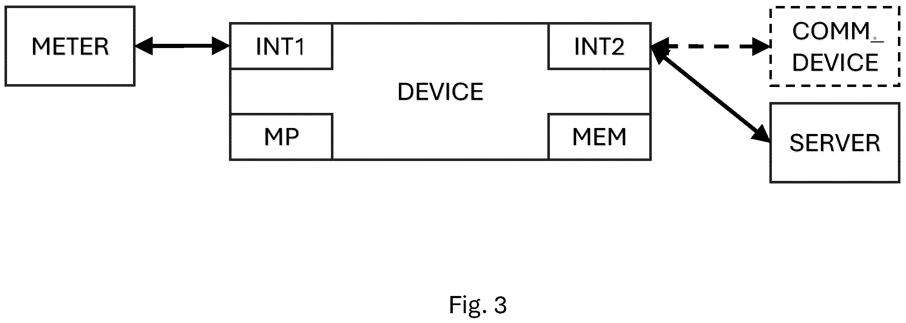

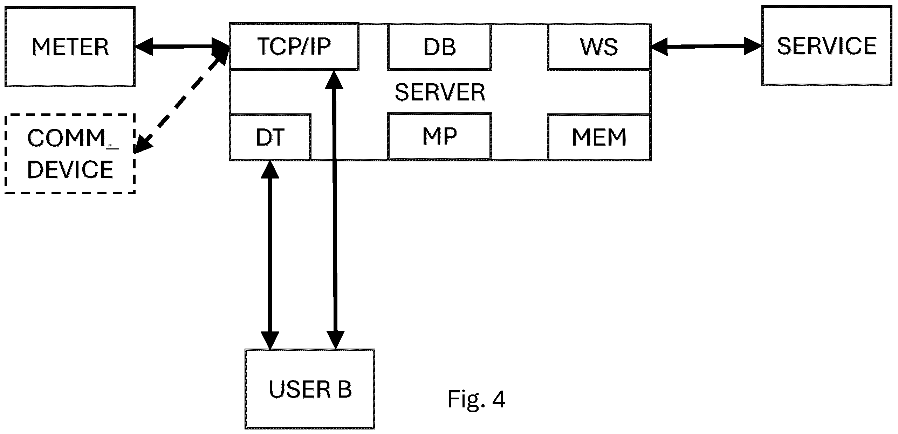

FIG. 3 and FIG. 4 schematically illustrate the main elements of the system according to the invention. In the second form of the invention, FIG. 3 shows the reading device, DEVICE.

The reading device, DEVICE, which communicates in step 101 with the metering apparatus, METER, measuring utility consumption and reads in step 102 the amount of consumed utility, and then transmits in step 103 the read data to the server or transmits in step 201 the read data to the intermediary communication device, COMM. DEVICE, comprises:
i. a microprocessor system, MP, for processing data on utility consumption,
ii. memory, MEM, for storing the database, DB, and performing computational operations,
iii. interface, INT1, for communication with the metering apparatus, METER,
iv. interface, INT2, for communication with the telecommunications network or with the intermediary communication device, COMM_DEVICE,
whereby the microprocessor system, MP, is configured to:
v. receiving, by the reading device, DEVICE, from the metering apparatus, METER, data on utility consumption,
vi. transmitting, by the reading device, DEVICE, to the master system, SERVER, or intermediary communication device, COMM _DEVICE, information on utility consumption.

FIG. 4 shows a simplified block diagram of the master device in the form of a remote server according to the invention. The server comprises:
a) a microprocessor system, MP, enabling the running of applications, performing calculations, communication with the database, DB, and with the TCP/IP network,
b) a TCP/IP network interface,
c) a server application, WS, enabling data exchange with the service, SERVICE, reading device, METER, or intermediary device, COMM _DEVICE, and the monitoring person, USER_B,
d) memory, MEM, for storing the database, server application, and performing computational operations.

The microprocessor system, while executing the server application, is configured to:
e) receiving, by the server, from the reading devices, METER, or intermediary communication device, COMM _DEVICE, read data on utility consumption,
f) transmitting, by the server, to the service, SERVICE, data on utility consumption,
g) transmitting, by the server, to the monitoring person, USER_B, via the telecommunications network, information on the well-being of the monitored person, USER A.

Preferably, the server is permanently connected to the telecommunications network. The monitoring person can receive messages about the well-being of the monitored person through an application on their mobile device, via email, or via SMS.

Each reading device connects recurrently to the utility metering apparatus and reads the amount of consumed utility or is permanently connected to it. The reading device connects to the server recurrently or on request from the server and transmits the amount of consumed utility, or transmits the amount of consumed utility continuously after obtaining a new measurement. Preferably, the device can encrypt the transmitted data to increase data transmission security.

In the third embodiment of this invention, a server is presented, comprising:
A. a microprocessor system enabling the running of applications, performing calculations, communication with the database, and with the TCP/IP network,
B. a TCP/IP network interface,
C. a server application enabling information exchange with the service, device, or intermediary device, and the monitoring user,
D. memory for storing the database, server application, and performing computational operations,
whereby the microprocessor system, while executing the server application, is configured to:
E. receiving, by the server, from the device or intermediary communication device, information on utility consumption,
F. transmitting, by the server, to the service, information on utility consumption,
G. transmitting, by the server, to the monitoring user, via the telecommunications network, information on the well-being of the monitored user.

Such a solution, unlike other known solutions, is simple and non-invasive, and therefore easily acceptable by monitored persons.

The proposed solution finds application, for example, in 1) monitoring the well-being of an elderly person based on electricity consumption using wireless Internet access, 2) monitoring the well-being of a person with a neurodegenerative disease (such as dementia) based on electricity and water consumption using a mobile network, 3) monitoring the well-being of a person with intellectual disabilities based on electricity and gas consumption using wireless Internet access. It should be clearly emphasized that the following examples are not intended to limit the scope of protection, but only to illustrate a few of the many possible applications of the subject invention. This invention can be applied wherever there is a need for discreet monitoring of the well-being of a person who, during normal functioning in her/his place of stay, consumes utilities such as electricity, water, or gas.

### Example 1

According to the method of the invention presented above in FIG. 1, the monitored elderly person, USER A, consumes electricity, MEDIUM, during her/his household activities such as using lighting, cooking, baking, washing, drying hair, and the like. The elderly person, with her/his consent, is monitored by her/his child, USER B, living in another city. The amount of consumed electricity, MEDIUM, is recorded in step 101 by the electricity consumption metering apparatus, METER. A beacon-type device, DEVICE, is attached to the metering apparatus, which communicates every 15 minutes in step 102 with the electricity metering apparatus, METER, via an optical port. The device, DEVICE, reads the amount of consumed energy in the last 15 minutes via the optical port and stores it in its memory. A smartphone-type communication device, COMM_DEVICE, communicates recurrently every 15 minutes in step 201 with the device, DEVICE, and reads the latest records in memory. It then transmits the records continuously in step 202 to the server, connecting to it using the TCP/IP protocol via a local Wi-Fi network connected to the Internet. The server stores the measurement results in a database. The server has previously created a model of electricity consumption by the monitored user, USER A. The application running on the server compares the current consumption with the electricity consumption model for the given user. If the consumption deviates from the model, for example, it is too high, too low, or has other atypical characteristics, the server in step 107 sends an appropriate message about the threatened well-being of the observed user, USER A, to the application installed on the monitoring user's smartphone, USER B. For example, disturbing information about the well-being of the monitored user may be increased electricity consumption at night, which may indicate, for example, sleep problems, or a lack of morning electricity consumption related to meal preparation, which may indicate depression or sudden illness.

### Example 2

According to the method of the invention presented previously in FIG. 2, a person with dementia, USER A, is remotely monitored. She/he is monitored, with her/his consent, by a relative, USER B. The user, USER A, consumes electricity, MEDIUM, during her/his household activities, such as using lighting, cooking, baking, washing, drying hair, and the like, the amount of which is recorded in step 101 by the electricity consumption metering apparatus, METER. She/he also consumes water, MEDIUM', the amount of which is recorded in step 101' by the water consumption metering apparatus, METER'. A beacon-type device, DEVICE, is attached to the electricity metering apparatus, which reads the amount of consumed electricity by reading the number of flashes of the LED located on the metering apparatus, METER. In this manner, the device, DEVICE, calculates the amount of consumed energy and stores it in its memory. The device, DEVICE, is equipped with an interface for connecting to the LTE mobile network, by means of which, every 15 minutes, it transmits in step 103 data on consumed electricity to the server. Similarly, a beacon-type device, DEVICE', is attached to the water metering apparatus, which reads the amount of consumed water via the optical interface of the metering apparatus, METER'. The device, DEVICE', is equipped with an interface for connecting to the LTE mobile network, by means of which, every 15 minutes, it transmits information about the consumed water to the server in step 103'. The server stores the measurement results of electricity and water consumption in the database. The server has previously created a model of electricity and water consumption by the monitored user, USER A. The application running on the server compares current consumption with the electricity and water consumption model for the given user. If the consumption deviates from the model, i.e., it is too high, too low, or has other atypical characteristics, the server in step 107 sends an appropriate message about the threatened well-being of the observed user, USER A, to the application installed on the monitoring user's smartphone, USERB. For example, disturbing information about the well-being of the monitored user, USER A, may be increased water consumption, which may indicate, for example, that the monitored person has forgotten to turn off the tap, or a lack of increased water consumption at certain times of the day, which may indicate neglect of hygiene.

### Example 3

According to the method of the invention presented previously in FIG. 2, a person with an intellectual disability, USER A, is remotely monitored. She/he is monitored, with her/his consent, by a parent, USER B. The apartment is equipped with a Wi-Fi access point, providing wireless Internet access. The user, USER A, consumes electricity, MEDIUM, during her/his household activities, such as using lighting, washing, drying hair, and the like, the amount of which is recorded in step 101 by the electricity consumption metering apparatus, METER. The monitored person, USER_A, also consumes gas, MEDIUM', which she/he uses for cooking, the amount of which is recorded in step 101' by the gas consumption metering apparatus, METER'. A beacon-type device, DEVICE, is attached to the electricity metering apparatus, METER, which reads the amount of consumed electricity via the RS-485 communication interface. In this manner, the device, DEVICE, calculates the amount of consumed electricity and stores it in its memory. The device, DEVICE, is equipped with an interface for connecting to the Wi-Fi network, by means of which, every 15 minutes, it transmits in step 103 information about the consumed electricity to the server. Similarly, a beacon-type device, DEVICE', is attached to the gas metering apparatus, which reads the amount of consumed gas via the isolated galvanic interface of the metering apparatus, METER'. The device, DEVICE', is equipped with an interface for connecting to the Wi-Fi network, by means of which, every 15 minutes, it transmits information about the consumed gas to the server in step 103'. The server stores the measurement results of electricity and gas consumption in the database. The server has previously created a model of electricity and gas consumption by the monitored user, USER A. The application running on the server compares current consumption with the electricity and gas consumption model for the monitored person, USER A. If the consumption deviates from the model, i.e., it is too high, too low, or has other atypical characteristics, the server in step 107 sends an appropriate message via the mobile telephone network in the form of an SMS about the threatened well-being of the monitored person, USER A, to the mobile phone number of the monitoring person, USER B. For example, disturbing information about the well-being of the monitored person, USER A, may be a lack of increased gas consumption during meal preparation times, which may indicate, for example, that the monitored person is neglecting the preparation of hot meals.

The proposed monitoring method does not require interference with installations in the user's apartment, does not require the user to wear any devices, such as a wristband, and does not require the installation of a camera or microphone in the user's place of residence, which may be perceived as violating privacy. The proposed method and system are suitable for use with various types of metering apparatuses intended for measuring the consumption of various utilities, such as electricity, water, or gas. These metering apparatuses may, but do not have to be smart; it is sufficient that they are equipped with an optical port, for example, compliant with IEC62056-21, a flashing diode, or another communication port, for example, a P1 port. It is possible to use the measurement of one utility or several utilities.

## Claims

1. A method of monitoring human well-being, using indirect recording of the activity of a monitored person through non-invasive reading of the consumption of at least one utility, **characterized in that** it comprises the steps of:
reading and transmitting read data on the amount of at least one consumed utility to a master unit, directly via a telecommunications network or using an intermediary communication device,
storing the read data in a dedicated utility consumption database, enabling the storage of current and historical data for analysis purposes,
performing, by analytical means, an analysis of the read data on the amount of at least one consumed utility in a given time,
transmitting, to a monitoring person via a telecommunications network, read data and/or results of the performed analysis of typical or atypical consumption of at least one mentioned utility by the monitored person.

2. The method according to claim 1, **characterized in that** the read utility is electricity and/or gas and/or water.

3. The method according to claim 1, **characterized in that** the analysis of the read data on the amount of consumed utilities in a given time consists of comparing the current consumption of each utility with its own previously read historical data.

4. The method according to claim 1, **characterized in that** the read data on the amount of consumed utilities is transmitted via a telecommunications network to a remote server directly or using an intermediary communication device, wherein the read data on the remote server is stored in a database, and analytical means operating on the server create a mathematical model of the consumption of said utilities based on historical data on their previous consumption, and based on the performed analysis of utility consumption, analysis results and optionally alerts are generated, which are transmitted via a telecommunications network to the monitoring person and presented on her/his terminal device.

5. The method according to claim 1 or 4, **characterized in that** the analytical means create different mathematical models for the monitored person, depending on different times of the year, in which the characteristics of utility consumption may differ.

6. The method according to claim 1, **characterized in that** reading the amount of consumed utility by the reading device is performed recurrently by connecting to the utility metering apparatus every predetermined time, for example every 15 minutes or on request from the server or intermediary device.

7. The method according to claim 1, **characterized in that** reading the amount of consumed utility by the reading device is performed online, and the data is transmitted after reading a measurement value that differs from the previously read value by a predetermined threshold value.

8. The method according to claim 1, **characterized in that** the non-invasive reading of the amount of consumed utility is performed by reading the metering apparatus readings using its optical port or by counting light pulses generated by an optical light source integrated with the utility metering apparatus.

9. The method according to claim 1, **characterized in that** reading the amount of consumed utility is performed by reading the metering apparatus readings using an isolated galvanic interface or a P1 port integrated with the utility metering apparatus.

10. The method according to claim 1, **characterized in that** the data transmission from the reading device is encrypted, preferably using the AES protocol, to increase data transmission security.

11. A system for monitoring human well-being, comprising at least one utility consumption metering apparatus, said utility is consumed by the monitored person, and at least one device configured for transmitting read data from said utility consumption metering apparatus to a master device, and a system for processing and storing read data, **characterized in that** a first reading device (DEVICE) is connected to a first utility metering apparatus (METER) in a non-invasive fashion and provides reading of data on the consumption of the first utility and transmission of the read data to a master device (SERVER) or via a communication device (COMM. DEVICE) to the master device (SERVER), and **in that** it has at least one second reading device (DEVICE) connected to a second utility metering apparatus (METER) in a non-invasive fashion, and providing reading of data on the consumption of the second utility and transmission of the read data to the master device (SERVER) or via a communication device (COMM. DEVICE) to the master device (SERVER), optionally also a third reading device (DEVICE) connected to a third utility metering apparatus (METER) in a non-invasive fashion, and providing reading of data on the consumption of the third utility and transmission of the read data to the master device (SERVER) or via a communication device (COMM. DEVICE) to the master device (SERVER), wherein all reading devices (DEVICE) have means for non-invasively reading or counting the amount of consumed utility and are connected to the master device (SERVER) in a manner ensuring data transmission, the master device (SERVER) has a system for processing the read data on the consumption of all read utilities, and memory means (MEM) configured to store read data on utility consumption, and means for performing computational operations, and means for transmitting to the monitoring person the results of the analysis on the well-being of the monitored person.

12. The system according to claim 11, **characterized in that** the first utility is electricity, the second utility is gas, and the third utility is water, or the first utility is electricity, the second utility is water, and the third utility is gas, or the first utility is water, the second utility is electricity, and the third utility is gas, or the first utility is water, the second utility is gas, and the third utility is electricity, or the first utility is gas, the second utility is electricity, and the third utility is water, or the first utility is gas, the second utility is water, and the third utility is electricity.

13. The system according to claim 11, **characterized in that** the master device is a server, the system for processing read data and the means for performing computational operations is a computer program running on the server, and a database storing read historical data on previous utility consumption is implemented in the memory means.

14. The system according to claim 11, **characterized in that** the reading device has means in the form of an optical port for non-invasively reading the amount of consumed utility or means for counting light pulses generated by an optical light source integrated with the utility metering apparatus.

15. The system according to claim 11, **characterized in that** the reading device has means in the form of an isolated galvanic interface or a P1 port integrated with the utility metering apparatus for non-invasively reading the amount of consumed utility.
